(19) Europäisches Patentamt
European Patent Office
Office européen des brevets

(11) **EP 4 309 693 B1**

(12) **EUROPEAN PATENT SPECIFICATION**

(45) Date of publication and mention
of the grant of the patent:
**10.12.2025 Bulletin 2025/50**

(21) Application number: **23186607.0**

(22) Date of filing: **20.07.2023**

(51) International Patent Classification (IPC):
**A61M 1/36** $^{(2006.01)}$

(52) Cooperative Patent Classification (CPC):
**A61M 1/3693;** A61M 1/265; A61M 2205/331

(54) **SYSTEM AND METHOD FOR CONTROLLING OUTLET CELL CONCENTRATION**

SYSTEM UND VERFAHREN ZUR STEUERUNG DER AUSTRITTSZELLENKONZENTRATION

SYSTÈME ET PROCÉDÉ DE RÉGULATION DE CONCENTRATION DE CELLULE DE SORTIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.07.2022 US 202263391415 P**

(43) Date of publication of application:
**24.01.2024 Bulletin 2024/04**

(73) Proprietor: **Fenwal, Inc.
Lake Zurich, IL 60047 (US)**

(72) Inventor: **KUSTERS, Benjamin E.
Lake Zurich, 60047 (US)**

(74) Representative: **Maikowski & Ninnemann
Patentanwälte Partnerschaft mbB
Postfach 15 09 20
10671 Berlin (DE)**

(56) References cited:
**US-A1- 2018 133 655     US-A1- 2019 290 832**

**Description**

Cross-Reference To Related Applications

This application claims the benefit of and priority of U.S. Provisional Patent Application Serial No. 63/391,415, filed July 22, 2022.

**Field of the Invention**

The present disclosure is directed to a separation system and an *ex vivo* method for separating a suspension of biological cells. The system monitors fluid flow in and out of a separator using optical measurements and making adjustments to the flow accordingly. The method adjusts the flow rate so that the outlet concentration is equivalent to a target outlet concentration.

**Background**

The use of devices for the separation of whole blood or its constituent components is widespread. Such devices commonly utilize centrifuges (that separate the cellular components based on their density) or filter membranes (that separate the cellular components based upon their size).

According to one approach, blood or blood components may be separated into its constituents through centrifugation. This requires that the blood or blood components be passed through a centrifuge. The centrifuge rotates the separation chamber of the disposable flow circuit during processing, causing the heavier (greater specific gravity) components of the whole blood or components in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Various ones of these components can be selectively removed from the whole blood by providing appropriately located channeling structures and outlet ports in the flow circuit.

While many blood separation systems and procedures have employed centrifugal separation principles, another common separation device is a spinning membrane separator. This type of device employs relatively rotating surfaces, at least one or which carries a porous membrane. Typically, the device employs an outer stationary housing and an internal spinning rotor covered by a porous membrane. A detailed description of an exemplary spinning membrane separator may be found in U.S. Patent No. 5,194,145. A further example is described in US2018/133655 A1. US2018/133655 A1 discloses a method for automated processing of a blood product, the method comprising providing a reusable separation apparatus controlled by a microprocessing unit, said apparatus configurable with settings and configured to associate with a disposable circuit comprising a separator and in communication with a source blood product having a first concentration and first volume. The apparatus and disposable circuit are configured to flow the source blood product into an inlet of the separator and separate supernatant of the source blood product from a first outlet of the separator into a filtrate container. The apparatus and disposable circuit are also configured to separate platelets and remaining supernatant from a second outlet of the separator into a retentate container, wherein the platelets and remaining supernatant in the retentate container have a second concentration greater than the first concentration and second volume less than the first volume.

In particular separation processes, such as a platelet separation process, the outlet cell concentration (platelet concentration) may impact the storage characteristics of the cells. In these particular processes it is desirable to ensure the outlet concentration is as close to a target outlet cell concentration as possible. Methods incorporating weight scales on the product bag(s) and ultrasonic flow sensors have proven less effective due to issues with accuracy and cost. Therefore, a need for a cost-effective solution to ensure that the fluid exiting the separator is at a target cell concentration is still needed.

**Summary**

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto. The invention is defined by the appended claims.

According to a first aspect, the present disclosure is directed to an *ex vivo* method for separating a suspension of biological cells at a target outlet cell concentration. The method is performed using a system comprising a separator with an inlet for introducing the suspension of biological cells into the separator and a first outlet for flowing cell concentrate from the separator. The system further comprises a first optical sensor on the inlet, a second optical sensor on the first outlet, and a controller. The method includes setting a target outlet concentration and target ratio of inlet concentration to outlet concentration, determining an inlet rate and an inlet concentration, and calculating an initial outlet rate based on the target ratio, measured inlet rate and measured inlet concentration. The method also includes setting the initial outlet rate for the separator and operating the separator at this outlet rate, performing an optical measurement at the first and second optical sensors, and comparing the ratio of the optical measurement at the first optical sensor to the optical measurement at the second optical sensor to the target ratio. Next, the method includes adjusting the separator to an adjusted outlet rate if the ratios are not equal by adjusting at least one pump associated with the separator, and performing previous steps until the ratio of the optical measurement at the first optical sensor to the optical measurement at the second optical sensor is equal to the target ratio, producing the target outlet cell concentration.

According to a second aspect, the present disclosure is directed to an *ex vivo* method for separating a suspension of biological cells at a target outlet cell concentration. The method is performed using a system comprising a separator with an inlet for introducing the suspension of biological cells into the separator and a first outlet for flowing cell concentrate from the separator. The system further comprises a first optical sensor on the inlet, a second optical sensor on the first outlet, and a controller. The method includes setting a target outlet concentration and target ratio of outlet concentration to inlet concentration, determining an outlet rate and an inlet concentration and calculating an initial inlet rate based on the target ratio, measured outlet rate and measured inlet concentration. The method further includes setting the initial inlet rate for the separator and operating the separator at this inlet rate, performing an optical measurement at the first and second optical sensors and comparing the ratio of the optical measurement at the second optical sensor to the optical measurement at the first optical sensor to the target ratio. Next, the method includes adjusting the separator to an adjusted inlet rate if the ratios are not equal by adjusting at least one pump associated with the separator, and performing previous steps until the ratio of the optical measurement at the second optical sensor to the optical measurement at the first optical sensor is equal to the target ratio, producing the target outlet cell concentration.

In a third aspect, the present disclosure is directed to a system for separating a suspension of biological cells. The system includes a fluid circuit comprising a separator with an inlet for introducing the suspension of biological cells into the separator and a first outlet for flowing cell concentrate from the separator. The system also includes a hardware component comprising a first pump for flowing the suspension of biological cells to the inlet of the separator, a second pump for flowing the cell concentrate from a first outlet of the separator, a first optical sensor associated with the first outlet, and a second optical sensor associated with the second outlet. The system further includes a programmable controller which is configured to set a target outlet concentration and set a target ratio of inlet concentration to outlet concentration, determine an inlet rate and an inlet concentration, and calculate an initial outlet rate based on the target ratio, measured inlet rate and measured inlet concentration. The controller is further configured to set the initial outlet rate for the separator and operate the separator at this outlet rate, perform an optical measurement at the first and second optical sensors and compare the ratio of the optical measurement at the first optical sensor to the optical measurement at the second optical sensor to the target ratio. The controller is also configured to adjust the separator to an adjusted outlet rate by adjusting the second pump if the ratios are not equal and perform previous steps until the ratio of the optical measurement at the first optical sensor to the optical measurement at the second optical sensor is equal to the target ratio, producing the target outlet cell concentration.

These and other aspects of the present subject matter are set forth in the following detailed description of the accompanying drawings.

## Brief Description of the Drawings

Fig. 1A is a schematic perspective view of a centrifugal separation chamber of the type that may be advantageously used in the system and method of the present disclosure.

Fig. 1B is a bottom perspective view of the fluid flow path through the centrifugal separation chamber of Fig. 1A.

Fig. 2 is a schematic cross-sectional view of a spinning membrane separator of the type that may be advantageously used in the system and method of the present disclosure.

Fig. 3 is a schematic representation of a system in accordance with the present disclosure which includes notations showing the inlet rate, outlet rate, inlet concentration, and outlet concentration.

Fig. 4 is a schematic representation of a system in accordance with the present disclosure which includes optical sensors.

Fig. 5 is a schematic representation of components of a separation system in accordance with the present disclosure.

Fig. 6 is a flow chart of the method of producing an outlet flow with a target outlet concentration.

**Detailed Description**

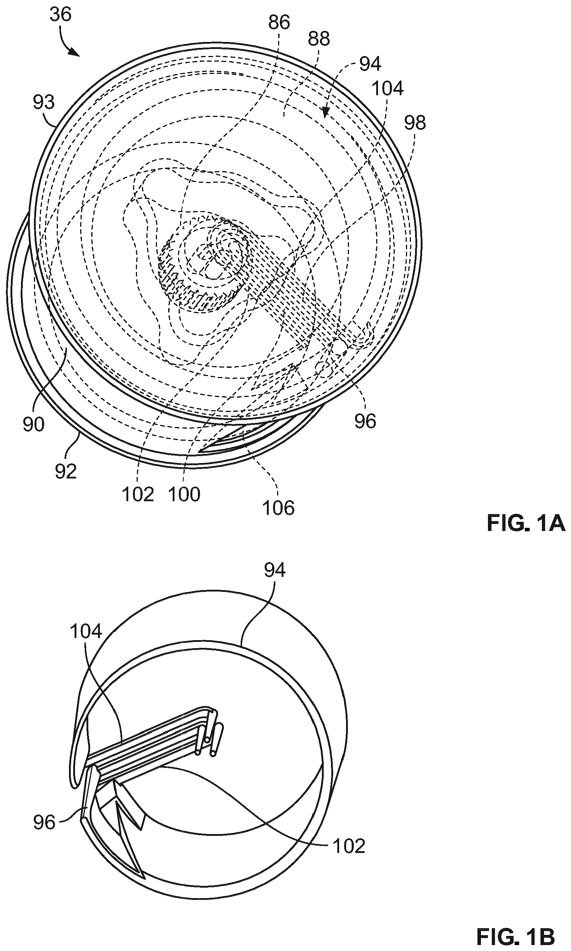

Figs. 1-5 show components of an automated blood or fluid separation system that embody various aspects of the present subject matter. While the system may be referred to herein as a "blood processing system" and specifically references blood components, it should be understood that systems according to the present disclosure can be used for processing a variety of biological fluids, which may include bodily fluids and non-bodily fluids.

"Biological fluid" includes without limitation blood and blood components, and "cell" or "biological cell" includes without limitation blood cells, such as red cells, white cells and platelets. By "automated," it is meant that the apparatus can be programmed to carry out the processing steps of a biological fluid processing method without substantial operator involvement. Of course, even in the automated system of the present disclosure, it will be understood that operator activity may be involved, including the loading of the disposable fluid circuits and entering processing parameters. Additional manual steps may be required as well. However, the reusable apparatus can process biological fluid through the disposable circuit(s) described below without substantial operator intervention.

The illustrated processing apparatus is typically capable of effecting the separation of a biological fluid that includes biological cells into two or more components or fractions. Thus, the reusable apparatus may generate conditions that allow for the separation of a biological fluid into selected components or fractions.

Generally speaking, the separation system includes a disposable fluid flow circuit with a spinning membrane separator or a centrifugal separator chamber, additional components that control fluid flow through the disposable flow circuit, and a controller, which governs the operation of the other components of the blood separation system to control the flow in and out of the separator.

The fluid flow circuit or flow set is intended to be a sterile, single use, disposable item. Before beginning a given blood processing and/or collection procedure, the operator mounts the various components of the fluid flow circuit into the blood separation system. The controller implements the procedure based upon preset protocols, taking into account other input from the operator. Upon completing the procedure, the operator removes the fluid flow circuit from association with the blood separation system. The portions of the fluid flow circuit holding the collected blood component or components (e.g., collection containers or bags) are removed and retained for storage, transfusion, or further processing. The remainder of the fluid flow circuit is removed and discarded.

The containers and the plastic tubing of the fluid circuit are made of conventional medical grade plastic that can be sterilized by sterilization techniques commonly used in the medical field such as, but not limited to, radiation or autoclaving. Plastic materials useful in the manufacture of containers and of the tubing in the circuits disclosed herein include plasticized poly(vinyl chloride). Other useful materials include acrylics. In addition, certain polyolefins may also be used.

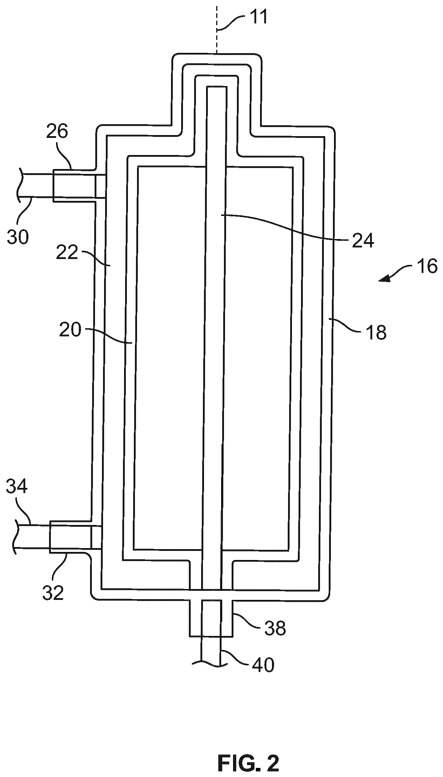

A variety of different disposable fluid flow circuits may be used in combination with the blood separation system, with the appropriate fluid flow circuit depending on the separation procedure to be carried out using the system. Generally speaking, though, the fluid flow circuit may include a cassette, to which the other components of the fluid flow circuit are connected by flexible tubing. The other components may include a plurality of fluid containers (for holding blood, a separated blood component, an intravenous fluid, or an additive solution, etc.,), one or more blood source access devices (e.g., a phlebotomy needle or a connector for accessing blood within a fluid container), and a separator. The separator may be a spinning membrane separator 16 (Fig. 2 ) and/or a centrifugal separation chamber 36 (Figs. 1A and 1B).

A fluid flow circuit may be provided with a centrifugal separation chamber 36 if platelets and/or white blood cells are to be separated. An exemplary centrifugal separation chamber 36 is shown in Figs. 1A and 1B.

The underside of the centrifugal separation chamber 36 includes a shaped receptacle 86 that is suitable for receiving an end of an umbilicus of a fluid flow circuit. A suitable receptacle 86 and the manner in which the umbilicus may cooperate with the receptacle 86 to deliver fluid to and remove fluid from the centrifugal separation chamber 36 are described in greater detail in U.S. Patent No. 8,075,468.

The illustrated centrifugal separation chamber 36 has radially spaced apart inner (low-g) and outer (high-g) side wall portions 88 and 90, a bottom or first end wall portion 92, and a cover or second end wall portion 93. The cover 93 comprises a simple flat part that can be easily welded or otherwise secured to the body of the centrifugal separation chamber 36. The wall portions 88 and 90, the bottom 92, and the cover 93 together define an enclosed, generally annular channel 94.

The (whole blood) inlet 96 communicating with the channel 94 is defined between opposing interior radial walls 98 and 100. One of the interior walls 98 joins the outer (high-g) wall portion 90 and separates the upstream and downstream ends of the channel 94. The interior walls 98 and 100 define the inlet passageway 96 of the centrifugal separation chamber 36 which, in one flow configuration, allows fluid to flow from the umbilicus 46 to the upstream end of the channel 94.

The illustrated centrifugal separation chamber 36 further includes first and second outlets 102 and 104, respectively, which may be defined by opposing surfaces of interior radial walls. Both the first and second outlets 102 and 104 extend radially inward from the channel 94. The first (platelet-rich plasma) outlet 102 extends radially inward from an opening which, in the illustrated embodiment, is located at the inner side wall portion 88, while the second (red blood cell) outlet 104 extends radially inward from an opening that is associated with the outer side wall portion 90. The illustrated first outlet 102 is positioned adjacent to the inlet 96 (near the upstream end of the channel 94), while the second outlet 104 may be positioned at the opposite, downstream end of the channel 94.

Blood flowed into the channel 94 separates into an optically dense layer RBC and a less optically dense layer PRP as the centrifugal separation chamber 36 is rotated about the rotational axis 38. The optically dense layer RBC forms as larger and/or heavier blood particles move under the influence of centrifugal force toward the outer (high-g) wall portion 90. The optically dense layer RBC will typically include red blood cells (and, hence, may be referred to herein as the "RBC layer") but, depending on the speed at which the centrifugal separation chamber 36 is rotated, other cellular components (e.g., larger white blood cells) may also be present in the RBC layer RBC.

The less optically dense layer PRP typically includes platelet-rich plasma (and, hence, will be referred to herein as the "PRP layer"). Depending on the speed at which the centrifugal separation chamber 36 is rotated and the length of time that the blood is resident therein, other components (e.g., smaller white blood cells and anticoagulant) may also be present in the PRP layer PRP.

The transition between the RBC layer RBC and the PRP layer PRP may be referred to as the interface INT. The location of the interface INT within the channel 94 of the centrifugal separation chamber 36 can dynamically shift during blood processing. The ideal or target interface INT may be experimentally determined, which may vary depending on any of a number of factors (e.g., the configuration of the centrifugal separation chamber 36, the rate at which the centrifugal separation chamber 36 is rotated about the rotational axis 38, etc.).

The blood separation system may include an interface monitoring system and a controller with an interface control module to monitor and, as necessary, correct the position of the interface INT.

Preferably, in accordance with the present disclosure, the fluid circuit includes a spinning membrane separator of the type described in U.S. Patent No. 5,194,145 and U.S. Patent No. 5,053,121.

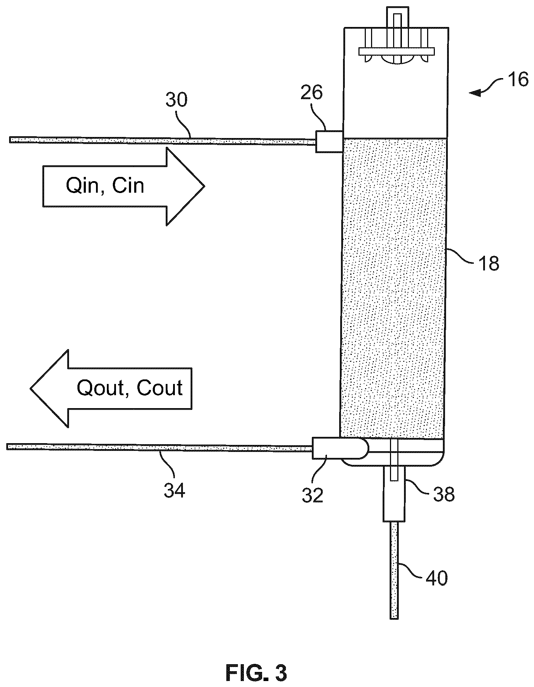

As shown in Figs. 2 and 3, the fluid circuit may include a spinning membrane separator 16 having a generally cylindrical housing 18 and an internal member 20 having a first side and a second side. A gap 22 is defined between the housing 18 and the first side of the filter membrane, while a flow path 24 is provided that is in fluid communication with the second side of the internal member 20.

The housing 18 includes an inlet 26 for introducing the suspension of biological cells into the gap 22 to which a source of the biological suspension to be separated is connected by a first tubing segment 30. The suspension to be separated may be contained in a reservoir, it could also be sourced directly from a donor by means of a donor access device (such as a phlebotomy needle) on the free end of the first tubing segment 30.

The housing 18 further includes a first outlet 32 in communication with the gap 22 for flowing retentate from the separator 16 through a second tubing segment 34 to a first collection container, and a second outlet 38 in communication with the flow path 24 on the second side of the filter membrane for flowing filtrate from the separator through a third tubing 40 segment to a second collection container. In one example, the suspension of biological cells may be whole blood, the retentate may be red blood cells and the filtrate may be plasma. In a second example, the suspension of biological cells may be platelet rich plasma, the retentate may be a platelet concentrate and the filtrate may be platelet free plasma.

The spacing between the housing and the spinner is sometimes referred to as the shear gap. The shear gap may be approximately 0.02 - 0.06 inches (0.05 - 0.15 cm) and may be of a uniform dimension along axis 11, for example, where the axis of the spinner and housing are coincident. The shear gap may also vary circumferentially for example, where the axis of the housing and spinner are offset.

The shear gap also may vary along the axial direction, for example preferably an increasing gap width in the direction. Such a gap width may range from about 0.02 to about 0.075 inches (0.05 - 0.19 cm). The gap width could be varied by varying the outer diameter of the rotor and/or the inner diameter of the facing housing surface. The gap width could change linearly or stepwise or in some other manner as may be desired. In any event, the width dimension of the gap is preferably selected so that at the desired relative rotational speed, Taylor-Couette flow, such as Taylor vortices, are created in the gap.

In the illustrated embodiment, the surface of the internal member 20 is at least partially, and is preferably substantially or entirely, covered by a cylindrical porous membrane. The membrane may have a nominal pore size between 0.8 and 10 microns ($\mu$m), for example. Membranes may be fibrous mesh membranes, cast membranes, track-etched membranes or other types of membranes that will be known to those of skill in the art. For example, in one embodiment, the membrane may have a polyester mesh (substrate) with nylon particles solidified thereon, thereby creating a tortuous path through which only certain sized components will pass. In an embodiment, the nylon membrane may have a pore size of approximately 0.8 $\mu$m and a thickness of approximately 150 $\mu$m or greater. Membranes of this type will typically retain all cellular components (e.g., red blood cells, white blood cells) and certain formed blood components, e.g., platelets. In another embodiment, the membrane may be made of a thin (approximately 10 $\mu$m thick) sheet of unsupported polycarbonate, for example, with a pore size of approximately 4.0 $\mu$m. In this embodiment, pores (holes) may be cylindrical and larger than those described above. The pores may be sized to allow small, formed components (e.g., platelets, microparticles, etc.) to pass, while the desired cells (e.g., white blood cells and larger red blood cells) are collected.

In a preferred embodiment, the housing 18 is stationary and the internal member 20 is a rotating spinner that is rotatable concentrically within the cylindrical housing 18. In such an embodiment, the housing 18 (or at least its upper and lower ends) are formed of non-magnetic material, while the internal member 20 includes an element (e.g., a metallic material) that interacts with a magnet of a spinning membrane separator drive unit to rotate the internal member 20 within the housing 18, as described above.

In embodiments utilizing a spinning membrane separator, the blood separation system may include a spinner support or spinning membrane separator drive unit for accommodating a generally cylindrical spinning membrane separator 16 of the fluid flow circuit. U.S. Patent No. 5,194,145 describes an exemplary spinning membrane separator drive unit that would be suitable for incorporation into the blood separation system, but it should be understood that the spinning membrane separator drive unit may be differently configured without departing from the scope of the present disclosure.

In embodiments utilizing a centrifugal chamber, the blood separation system may also include a centrifugal separator. The particular configuration and operation of the centrifugal separator depends upon the particular configuration of the centrifugal separation chamber 36 of the fluid flow circuit. In one embodiment, the centrifugal separator is similar in structure and operation to that of the ALYX system manufactured by Fenwal, Inc. of Lake Zurich, Illinois, which is an affiliate of Fresenius Kabi AG of Bad Homburg, Germany, as described in greater detail in U.S. Patent No. 8,075,468.

The system may also include a plurality of pumps to cause fluid to flow through the fluid flow circuit. The pumps may be differently or similarly configured and/or function similarly or differently from each other. The pumps may be configured as peristaltic pumps, which may be generally configured as described in U.S. Patent No. 5,868,696. Particularly, a first pump may be associated with an inlet of a separation device and a second pump may be associated with an outlet of the separation device. In embodiments with a spinning membrane separator, the outlet may be the retentate outlet 32.

The system may include weight scales for product bags and may be provided with a plurality of hooks or supports that may support various components of the fluid flow circuit or other suitably sized and configured objects.

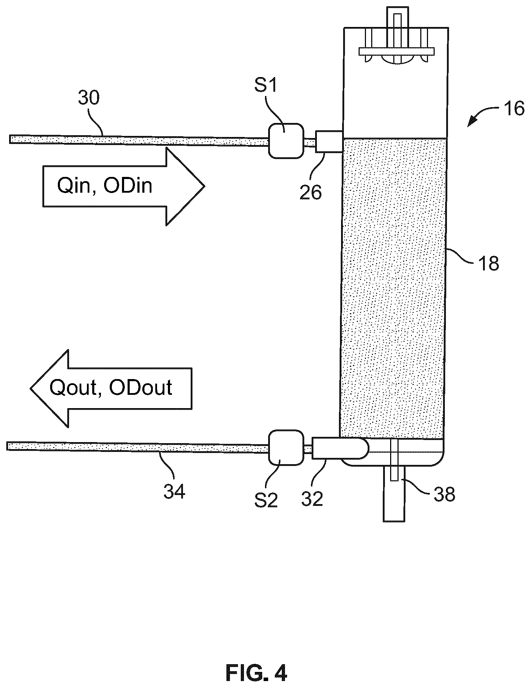

As shown in Fig. 4, the separation system may include an inlet sensor S1 for determining one or more properties of a fluid flowing into a separator and an outlet sensor S2 for determining one or more properties of the fluid flowing out of the separator. Although the example is shown in relation to a spinning membrane separator, the inlet and outlet sensors may be similarly applied to the centrifugal chamber or a centrifuge separator. If the fluid flowing into the spinning membrane separator 16 is whole blood the inlet sensor S1 may be configured to determine the hematocrit of the blood flowing into the spinning membrane separator 16. In one embodiment, the inlet sensor S1 and the outlet sensor S2 may be configured to determine the concentration of cellular blood components in the fluid. For instance, if the fluid flowing into the spinning membrane separator 16 is platelet-rich plasma, the inlet sensor S1 and outlet sensor S2 may be configured to determine the platelet concentration of platelet-rich plasma flowing into the spinning membrane separator 16.

The inlet sensor S1 and outlet sensor S2 may be optical sensors, which make an optical measurement. The optical measurements may include fluid absorption coefficient or scattering coefficient. Additionally, optical measurements may include transmission and reflection values. Also, the sensors may measure the optical density of the fluid. Depending on the specific type of fluid passing through the separator, the density may increase between the inlet sensor S1 and the outlet sensor S2. The optical sensor may compute the optical density by measuring light transmission, reflection, or a combination of the two or use the measured transmission or reflection values in comparison. The method is not limited by the optical sensing method for determining optical density. The controller may receive signals from the sensors that are indicative of the optical properties of fluid flowing into and out of the spinning membrane separator 16 and use the signals to optimize the separation procedure based upon that property or properties. In one embodiment, separated plasma flows through the tubing, in which case the inlet sensor S1 and outlet sensor S2 may be configured to determine the concentration of cellular blood components in the plasma. This may be done using an optical monitor of the type described in U.S. Patent No. 8,556,793 or by any other suitable device and/or method.

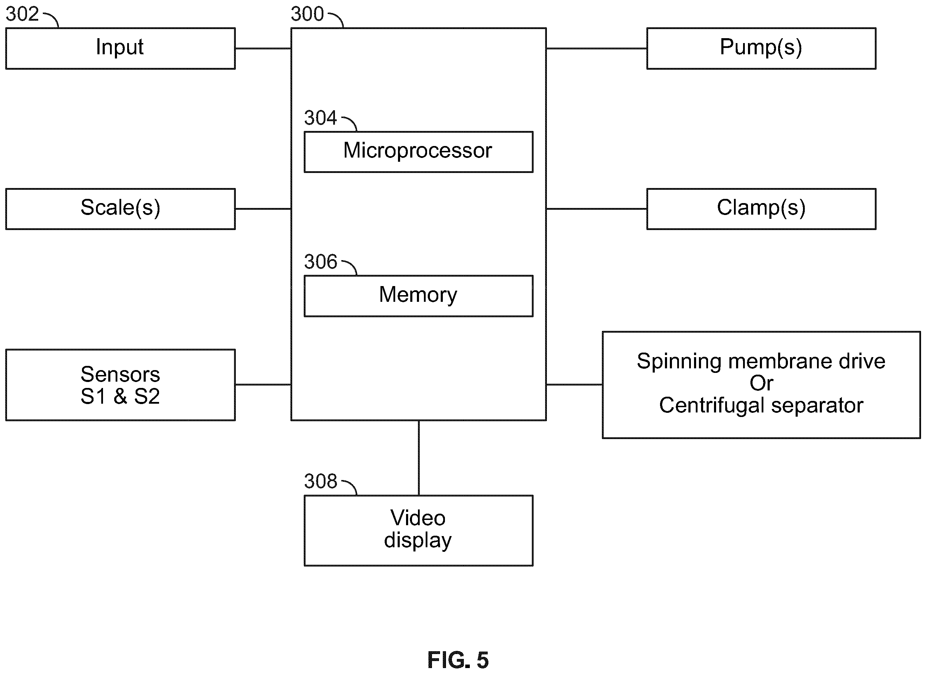

According to an aspect of the present disclosure, the blood separation system includes a controller 300, schematically shown in Fig. 5, which is suitably configured and/or programmed to control operation of the separation system. The controller 300 may include a microprocessor 304 (which, in fact may include multiple physical and/or virtual processors). According to other embodiments, the controller 300 may include one or more electrical circuits designed to carry out the actions described herein. In fact, the controller 300 may include a microprocessor and other circuits or circuitry. In addition, the controller 300 may include one or more memories 306. The instructions by which the microprocessor 304 is programmed may be stored on the memory 306 associated with the microprocessor 304, which memory/memories 306 may include one or more tangible non-transitory computer readable memories, having computer executable instructions stored thereon, which when executed by the microprocessor 304, may cause the microprocessors 304 to carry out one or more actions as described below.

As is also illustrated in Fig. 5, the controller 300 may be coupled to one or more of the structures described above, for example to receive information (e.g., in the form of signals) from these structures or to provide commands (e.g., in the form of signals) to these structures to control the operation of the structures. As illustrated, the controller 300 may be coupled to scales, the sensors S1 and S2 and the at least one input 302 to receive information from those devices. Additionally, the controller 300 may be coupled to pumps, clamps, and the drive or centrifuge separator to provide commands to those devices to control their operation. The controller 300 may be directly electrically connected to these structures to be coupled to them, or the controller 300 may be directly connected to other intermediate equipment that is directly connected to these structures to be coupled to them.

The at least one input 302 may include a number of different devices according to the embodiments described herein. For example, the input 302 could include a keyboard or keypad by which a user may provide information and/or instructions to the controller 300. Alternatively, the input 302 may be a touch screen, such as may be used in conjunction with a video display 308. The input could also include a reader or scanner, such as a barcode reader or scanner or an RFID reader. The assembly of the input/touch screen 302 and video display 308 may be one of the afore-mentioned structures to which the controller 300 is coupled from which the controller 300 receives information and to which the controller 300 provides commands. According to still other embodiments, the input 302 may be in the form of computer equipment that permits the blood separation system including the controller 300 to communicate (whether via wires, cables, etc. or wirelessly) with other systems over a local network, or with other cell processing systems or other computer equipment (e.g., a server) over local networks, wide area networks, or the Internet. According to such an embodiment, the input may include an internal transmitter/receiver device.

The controller is configured and/or programmed to execute at least one fluid processing application but, more advantageously, is configured and/or programmed to execute a variety of different fluid processing applications.

More particularly, in carrying out any one of these blood processing applications, the controller is configured and/or programmed to control the flow of a fluid through a separator and adjusts the speed of the flow into and out of the separator. This may include instructing the spinning membrane separator drive unit or the centrifugal separator to operate at a particular rotational speed and instructing a first pump to convey fluid through the inlet of the separator at a particular flow rate and instructing a second pump to convey fluid out of the outlet of the separator at a particular flow rate. Any known method of adjusting speed of the flow into and out of the separator may be used. Hence, while it may be described herein that a particular component of the blood separation system performs a particular function, it should be understood that that component is being controlled by the controller to perform that function.

Before, during, and after a procedure, the controller may receive readings from the sensors S1 and S2 and may have to adjust based on these readings.

For example, the controller may instruct one of the pumps to cause blood to flow into the separator at a different rate and/or for a separated blood component to be removed from the separator at a different rate and/or for the drive unit to be spun at a different speed.

Such control typically occurs regardless of whether the blood originates from a container or directly from a donor, and regardless of whether the components are directed into storage containers or returned to a donor or another living recipient.

The system, specifically the controller, may act to regulate the concentration of cellular content exiting the separator. A governing equation of separation by any separator is the mass balance relationship, which may be calculated as follows:

$$C_{out}Q_{out} = C_{in}Q_{in} \qquad \text{[Equation 1]}$$

$C_{out}$ is concentration of cellular content exiting the separator, $Q_{out}$ is flow rate of the fluid exiting the separator, $C_{in}$ is the concentration of the cellular content entering the separator, and $Q_{in}$ is the flow rate of the fluid entering the separator. Equation 1 can be rearranged to solve for any parameter of interest. If the fluid inlet concentration and flow rate ($C_{in}$ & $Q_{in}$) are known and a target outlet concentration ($C_{out}$) is desired, then the relationship can be rearranged to solve for the outlet fluid flow rate ($Q_{out}$) required to obtain a desired target outlet concentration ($C_{out}$) based on the known inlet concentration and flow rate ($C_{in}$ & $Q_{in}$), which may be calculated as follows:

$$Q_{out} = (C_{in}Q_{in})/C_{out} \qquad \text{[Equation 2]}$$

If all parameters are accurate (ex: pumps are achieving targeted flow rates) then the system will obtain the desired/targeted outlet concentration. Reversely, if the fluid inlet concentration and outlet flow rate are known and a target outlet concentration is desired, then the relationship can be rearranged to solve for the inlet fluid flow rate required to obtain a desired target outlet concentration based on the known inlet concentration and outlet flow rate, which may be calculated as follows:

$$Q_{in} = (C_{out}Q_{out})/C_{in} \qquad \text{[Equation 3]}$$

Again, if all parameters are accurate (ex: pumps are achieving targeted flow rates) then the system will obtain the desired/targeted outlet concentration.

Once a procedure begins, optical measurements, such as the optical density, of the inlet may be measured by optical sensor S1 on the inlet line and the optical density of the outlet may be measured by optical sensor S2 on the outlet line. The optical sensor may compute the optical density by measuring light transmission, reflection, or a combination of the two.

Using the measured optical densities, an outlet concentration can be calculated. There may be any of a number of errors which can cause an inaccuracy in the outlet concentration that is produced. Inaccuracy of the actual outlet concentration compared to the target outlet concentration is common and most often due to one or more of the assumed values being incorrect, such as pump driven flow rates not equaling target flow rate values.

In order to correct the system to achieve a target outlet concentration, the controller can adjust the inlet or outlet flow rates. This may be accomplished in a number of ways. Typically, the control of fluid into and out of the spinning membrane device has been accomplished by applying a first pump to the inlet line of the separator to supply a fluid source, and a second pump applied to either the outlet line for the retentate or the outlet line for the filtrate and adjusting the pump speed. The controller can also adjust the drive unit or centrifuge. The inlet or outlet flow rates may be adjusted by an amount calculated after measuring the $OD_{in}$ and $OD_{out}$.

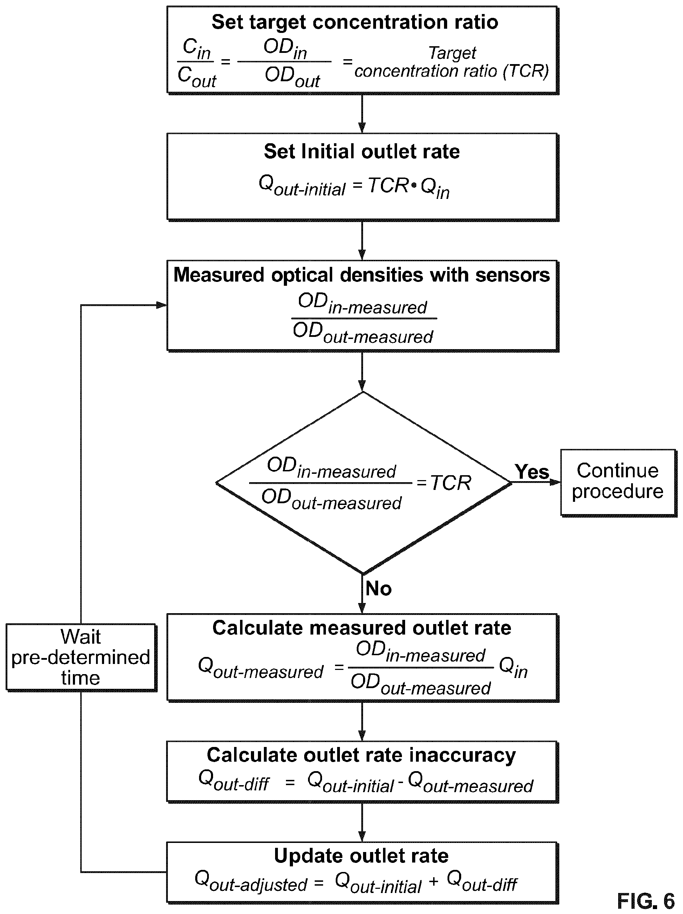

Fig. 6 shows a flow chart of the process of achieving a target outlet concentration by adjusting an outlet rate. In order to achieve a target cellular concentration exiting the spinner a specific concentration ratio must be achieved by reducing the volume of the fluid through filtration, by the following equation:

$$(C_{in}/C_{out})= \text{ target concentration ratio [Equation 4]}$$

The concentration ratio should be equal to the ratio of the Optical Density of the inlet ($OD_{in}$) to the optical density of the outlet ($OD_{out}$) shown in the following equation:

$$(C_{in}/C_{out})= \text{ target concentration ratio } =(OD_{in}/OD_{out}) \qquad \text{[Equation 5]}$$

Considering there is a known change in the optical density (OD) of the fluid with respect to a change in cellular concentration (optical density increases as concentration increases) and assuming (as suggested by known data) the ratio equals the concentration ratio, the relationships above can be reconstructed in terms on optical density (OD) as following:

$$OD_{out}Q_{out}= OD_{in}Q_{in} \quad \text{[Equation 6]}$$

The system next needs to calculate the optimal outlet rate given the target concentration ratio and the inlet rate in the following equation:

$$Q_{out\text{-}initial} = \text{ target concentration ratio } * Q_{in} \text{ [Equation 7]}$$

Then, once the procedure begins the measured optical density ratio ($OD_{in\text{-}measured}/ OD_{out\text{-}measured}$), which may not equal the target concentration/optical density ratio, can be used to determine the measured outlet rate ($Q_{out\text{-}measured}$):

$$Q_{out\text{-}measured} = (OD_{in\text{-}measured}/ OD_{out\text{-}measured}) * Q_{in} \quad \text{[Equation 8]}$$

Once the measured outlet rate is calculated it is compared to the initial outlet rate to determine any inaccuracies present in the pump:

$$Q_{out\text{-}diff}= Q_{out\text{-}initial} - Q_{out\text{-}measured} \text{ [Equation 9]}$$

Then, the system will then adjust the outlet rate ($Q_{out\text{-}adjusted}$) in an attempt to move the measured optical density ratio ($OD_{in\text{-}measured}/ OD_{out\text{-}measured}$), equal to the targeted optical density ratio/concentration ratio established:

$$Q_{out\text{-}adjusted}= Q_{out\text{-}initial} + Q_{out\text{-}diff} \text{ [Equation 10]}$$

It may be possible that the actual inlet rate $Q_{in}$ does not equal the target rate that is held constant in the equations or for the adjusted outlet rate to still be inaccurate. To account for these inaccuracies the method works in a loop to ensure an outlet rate is achieved that produces a measured optical density ratio equal to the target optical density ratio. Once the measured ratio equals the target ratio, the outlet concentration will equal the desired/target concentration regardless of the actual flow rates values.

Instead of adjusting the outlet rate to obtain the desired outlet concentration, the inlet rate can be adjusted in a similar manner. Since the inlet rate is being adjusted the target concentration ratio will instead be calculated as follows:

$$(C_{out}/C_{in})= \text{ target concentration ratio [Equation 11]}$$

The concentration ratio should be equal to the ratio of the optical density of the outlet ($OD_{out}$) to the optical density of the inlet ($OD_{in}$) shown in the following equation:

$$(C_{out}/C_{in})= \text{ target Concentration ratio }=(OD_{out}/OD_{in}) \quad \text{[Equation 12]}$$

The optimal inlet rate given the target concentration ratio and the outlet rate in the following equation:

$$Q_{in\text{-}initial} = \text{ target concentration ratio} * Q_{out} \text{ [Equation 13]}$$

Then, once the procedure begins the measured optical density ratio ($OD_{out\text{-}measured}/ OD_{in\text{-}measured}$), which may not equal the target concentration/optical density ratio, can be used to determine the measured inlet rate ($Q_{in\text{-}measured}$):

$$Q_{in\text{-}measured} = (OD_{out\text{-}measured}/ OD_{in\text{-}measured}) * Q_{out} \text{ [Equation 14]}$$

Once the measured inlet rate is calculated it is compared to the initial inlet rate to determine any inaccuracies present in the system:

$$Q_{in\text{-}diff}= Q_{in\text{-}initial} - Q_{in\text{-}measured} \text{ [Equation 14]}$$

Then, the system will then adjust the inlet rate $Q_{in\text{-}adjusted}$ in an attempt to move the measured optical density ratio ($OD_{out\text{-}measured}/ OD_{in\text{-}measured}$), equal to the targeted optical density ratio/concentration ratio established:

$$Q_{in\text{-}adjusted}= Q_{in\text{-}initial} + Q_{in\text{-}diff} \text{ [Equation 15]}$$

Example:

Example 1: $Q_{in}$=30 mL/min; $C_{in}$=250 $e^3$/uL; desired $C_{out}$=1500$e^3$/uL

$$(C_{in}/C_{out})= (250/1500)= 1/6$$

$$1/6= \text{Target Concentration Ratio}$$

$$Q_{out\text{-}initial} =\text{Target Concentration Ratio} * Q_{in} = (1/6) \times 30mL/min= 5mL/min$$

The system is set to an outlet rate of 5mL/min. After some running time, optical densities are measured, and the actual ration is determined. $OD_{in\text{-}measured}$=5 OD units; $OD_{out\text{-}measured}$=37.5 OD units. The $Q_{out\text{-}measured}$ can be calculated:

$$Q_{out\text{-}measured} = (OD_{in\text{-}measured}/ OD_{out\text{-}measured}) * Q_{in} = (5/37.5) * 30mL/min = 4mL/min$$

The measured optical density ratio does not equal the target value of (1/6). The system is, therefore, over-concentrating the cells and it is assumed that the $Q_{out}$ is pumping too slow (4mL/min) instead of (5 mL/min). The $C_{out}$ is:

$$C_{out}= (C_{in}/Q_{in})/Q_{out} = (250 * 30)/ 4 = 1875e^3/uL$$

The outlet concentration is 1875e3/uL versus the desired 1500e$^3$/uL and the outlet rate will have to be adjusted accordingly. The outlet rate difference is calculated:

$$Q_{out-diff} = Q_{out-initial} - Q_{out-measured} = 5 \text{ mL/min} - 4 \text{ mL/min} = 1 \text{ mL/min}$$

This amount is then added to the initial outlet rate and the new adjusted outlet rate for the system is calculated:

$$Q_{out-adjusted} = Q_{out-initial} + Q_{out-diff} = 5 \text{ mL/min} + 1 \text{ mL/min} = 6 \text{ mL/min}$$

The system (controller) will then adjust the outlet rate to 6 mL/min and after allowing time to correct, take additional optical measurements to determine if the concentration is on target. The system will keep testing and adjusting as needed to achieve the target concentration.

Thus, an improved fluid separation system and method have been provided. The system and method should provide for greater accuracy in controlling the outlet concentration from the separator by utilizing inlet and outlet optical sensors and adjusting components of the system to regulate the flow in and out of the separator.

**Claims**

1. An ex-vivo method for separating a suspension of biological cells at a target outlet cell concentration using a system comprising a separator with an inlet for introducing the suspension of biological cells into the separator and a first outlet for flowing cell concentrate from the separator, a first optical sensor on the inlet, a second optical sensor on the first outlet, at least one pump associated with the separator, and a controller, the method comprising:

   a. setting a target outlet concentration and target ratio of inlet concentration to outlet concentration;
   b. determining an inlet rate and an inlet concentration;
   c. calculating an initial outlet rate based on the target ratio, measured inlet rate and measured inlet concentration;
   d. setting the initial outlet rate for the separator and operating the separator at this outlet rate;
   e. performing an optical measurement at the first and second optical sensors;
   f. comparing the ratio of the optical measurement at the first optical sensor to the optical measurement at the second optical sensor to the target ratio;
   g. adjusting the separator to an adjusted outlet rate if the ratios are not equal by adjusting the at least one pump associated with the separator; and
   h. performing steps e-g until the ratio of the optical measurement at the first optical sensor to the optical measurement at the second optical sensor is equal to the target ratio, producing the target outlet cell concentration.

2. The method of separating of claim 1, wherein the separator is a spinning membrane separator.

3. The method of separating of claim 2, wherein the separator includes a first and second outlet, the first outlet for flowing retentate from the separator.

4. The method of separating of claim 1, wherein the separator includes a centrifugal chamber.

5. The method of separating of any of the preceding claims, wherein the optical measurement is optical density.

6. The method of separating of any of the preceding claims, wherein the adjusting the at least one pump associated with the separator includes altering a pump associate with the first outlet to adjust the outlet rate.

7. The method of separating of any of the preceding claims, wherein the adjusting includes calculating the difference between the measured outlet rate and the initial outlet rate and adding the difference to the initial outlet rate to get the adjusted outlet rate.

8. An ex-vivo method for separating a suspension of biological cells at a target outlet cell concentration using a system comprising a separator with an inlet for introducing the suspension of biological cells into the separator and a first outlet for flowing cell concentrate from the separator, a first optical sensor on the inlet, a second optical sensor on the first outlet, at least one pump associated with the separator, and a controller, the method comprising:

a. setting a target outlet concentration and target ratio of outlet concentration to inlet concentration;

b. determining an outlet rate and an inlet concentration;

c. calculating an initial inlet rate based on the target ratio, measured outlet rate and measured inlet concentration;

d. setting the initial inlet rate for the separator and operating the separator at this inlet rate;

e. performing an optical measurement at the first and second optical sensors;

f. comparing the ratio of the optical measurement at the second optical sensor to the optical measurement at the first optical sensor to the target ratio;

g. adjusting the separator to an adjusted inlet rate if the ratios are not equal by adjusting the at least one pump associated with the separator; and

h. performing steps e-g until the ratio of the optical measurement at the second optical sensor to the optical measurement at the first optical sensor is equal to the target ratio, producing the target outlet cell concentration.

9. The method of separating of claim 8, wherein the separator is a spinning membrane separator.

10. The method of separating of claim 9, wherein the separator includes a first and second outlet, the first outlet for flowing retentate from the separator.

11. The method of separating of claim 8, wherein the separator includes a centrifugal chamber.

12. The method of separating of any of claims 8-11, wherein the optical measurement is optical density.

13. The method of separating of any of claims 8-12, wherein the adjusting the at least one pump associated with the separator includes adjusting a pump associated with the first inlet to adjust the inlet rate.

14. The method of separating of any of claims 8-13, wherein the adjusting includes calculating the difference between the measured inlet rate and the initial inlet rate and adding the difference to the initial inlet rate to get the adjusted inlet rate.

15. A system for separating a suspension of biological cells comprising:

i. a fluid circuit comprising a separator with an inlet for introducing the suspension of biological cells into the separator and a first outlet for flowing cell concentrate from the separator;

ii. a hardware component comprising a first pump for flowing the suspension of biological cells to the inlet of the separator, a second pump for flowing the cell concentrate from a first outlet of the separator, a first optical sensor associated with the first outlet, a second optical sensor associated with the second outlet, and a programmable controller configured to

a. set a target outlet concentration and set a target ratio of inlet concentration to outlet concentration;

b. determine an inlet rate and an inlet concentration;

c. calculate an initial outlet rate based on the target ratio, measured inlet rate and measured inlet concentration;

d. set the initial outlet rate for the separator and operate the separator at this outlet rate;

e. perform an optical measurement at the first and second optical sensors;

f. compare the ratio of the optical measurement at the first optical sensor to the optical measurement at the second optical sensor to the target ratio;

g. adjust the separator to an adjusted outlet rate by adjusting the second pump if the ratios are not equal; and

h. perform steps e-g until the ratio of the optical measurement at the first optical sensor to the optical measurement at the second optical sensor is equal to the target ratio, producing the target outlet cell concentration.

**Patentansprüche**

1. Ex-vivo-Verfahren zum Trennen einer Suspension biologischer Zellen bei einer Zielaustrittszellkonzentration unter Verwendung eines Systems, umfassend einen Abscheider mit einem Einlass zum Einführen der Suspension biologischer Zellen in den Abscheider und einem ersten Auslass zum Strömen des Zellkonzentrats aus dem Abscheider, einen ersten optischen Sensor an dem Einlass, einen zweiten optischen Sensor an dem ersten Auslass, mindestens eine Pumpe, die dem Abscheider zugeordnet ist, und eine Steuerung, wobei das Verfahren Folgendes umfasst:

a. Einstellen einer Ziel-Auslasskonzentration und eines Zielverhältnisses zwischen Einlasskonzentration und Auslasskonzentration;

b. Bestimmen einer Einlassrate und einer Einlasskonzentration;

c. Berechnen einer Anfangsauslassrate anhand des Zielverhältnisses, der gemessenen Einlassrate und der gemessenen Einlasskonzentration;

d. Einstellen der Anfangsauslassrate für den Abscheider und Betreiben des Abscheiders mit dieser Auslassrate;

e. Durchführen einer optischen Messung an dem ersten und zweiten optischen Sensor;

f. Vergleichen des Verhältnisses zwischen der optischen Messung an dem ersten optischen Sensor und der optischen Messung an dem zweiten optischen Sensor mit dem Zielverhältnis;

g. Anpassen des Abscheiders auf eine angepasste Auslassrate, wenn die Verhältnisse nicht gleich sind, durch Anpassen der mindestens einen Pumpe, die dem Abscheider zugeordnet ist; und

h. Durchführen der Schritte e-g, bis das Verhältnis zwischen der optischen Messung an dem ersten optischen Sensor und der optischen Messung an dem zweiten optischen Sensor gleich dem Zielverhältnis ist, wodurch die Zielaustrittszellkonzentration produziert wird.

2. Trennverfahren nach Anspruch 1, wobei der Abscheider ein Drehmembranabscheider ist.

3. Trennverfahren nach Anspruch 2, wobei der Abscheider einen ersten und einen zweiten Auslass beinhaltet, wobei der erste Auslass dazu dient, Retentat aus dem Abscheider strömen zu lassen.

4. Trennverfahren nach Anspruch 1, wobei der Abscheider eine Zentrifugalkammer beinhaltet.

5. Trennverfahren nach einem der vorhergehenden Ansprüche, wobei die optische Messung die optische Dichte ist.

6. Trennverfahren nach einem der vorhergehenden Ansprüche, wobei das Anpassen der mindestens einen Pumpe, die dem Abscheider zugeordnet ist, das Ändern einer Pumpe umfasst, die dem ersten Auslass zugeordnet ist, um die Auslassrate anzupassen.

7. Trennverfahren nach einem der vorhergehenden Ansprüche, wobei das Anpassen das Berechnen der Differenz zwischen der gemessenen Auslassrate und der Anfangsauslassrate und das Addieren der Differenz zu der Anfangsauslassrate beinhaltet, um die angepasste Auslassrate zu erhalten.

8. Ex-vivo-Verfahren zum Trennen einer Suspension biologischer Zellen bei einer Zielaustrittszellkonzentration unter Verwendung eines Systems, umfassend einen Abscheider mit einem Einlass zum Einführen der Suspension biologischer Zellen in den Abscheider und einem ersten Auslass zum Strömen des Zellkonzentrats aus dem Abscheider, einen ersten optischen Sensor an dem Einlass, einen zweiten optischen Sensor an dem ersten Auslass, mindestens eine Pumpe, die dem Abscheider zugeordnet ist, und eine Steuerung, wobei das Verfahren Folgendes umfasst:

a. Einstellen einer Ziel-Auslasskonzentration und eines Zielverhältnisses zwischen Auslasskonzentration und Einlasskonzentration;

b. Bestimmen einer Auslassrate und einer Einlasskonzentration;

c. Berechnen einer Anfangseinlassrate anhand des Zielverhältnisses, der gemessenen Auslassrate und der gemessenen Einlasskonzentration;

d. Einstellen der Anfangseinlassrate für den Abscheider und Betreiben des Abscheiders mit dieser Einlassrate;

e. Durchführen einer optischen Messung an dem ersten und zweiten optischen Sensor;

f. Vergleichen des Verhältnisses zwischen der optischen Messung an dem zweiten optischen Sensor und der optischen Messung an dem ersten optischen Sensor mit dem Zielverhältnis;

g. Anpassen des Abscheiders auf eine angepasste Einlassrate, wenn die Verhältnisse nicht gleich sind, durch Anpassen der mindestens einen Pumpe, die dem Abscheider zugeordnet ist; und

h. Durchführen der Schritte e-g, bis das Verhältnis zwischen der optischen Messung an dem zweiten optischen Sensor und der optischen Messung an dem ersten optischen Sensor gleich dem Zielverhältnis ist, wodurch die Zielaustrittszellkonzentration produziert wird.

9. Trennverfahren nach Anspruch 8, wobei der Abscheider ein Drehmembranabscheider ist.

10. Trennverfahren nach Anspruch 9, wobei der Abscheider einen ersten und einen zweiten Auslass beinhaltet, wobei der erste Auslass dazu dient, Retentat aus dem Abscheider strömen zu lassen.

**11.** Trennverfahren nach Anspruch 8, wobei der Abscheider eine Zentrifugalkammer beinhaltet.

**12.** Trennverfahren nach einem der Ansprüche 8-11, wobei die optische Messung die optische Dichte ist.

**13.** Trennverfahren nach einem der Ansprüche 8-12, wobei das Anpassen der mindestens einen Pumpe, die dem Abscheider zugeordnet ist, das Anpassen einer Pumpe umfasst, die dem ersten Einlass zugeordnet ist, um die Einlassrate anzupassen.

**14.** Trennverfahren nach einem der Ansprüche 8-11, wobei das Anpassen das Berechnen der Differenz zwischen der gemessenen Einlassrate und der Anfangseinlassrate und das Addieren der Differenz zu der Anfangseinlassrate beinhaltet, um die angepasste Einlassrate zu erhalten.

**15.** System zum Trennen einer Suspension biologischer Zellen, das Folgendes umfasst:

i. einen Fluidkreislauf, umfassend einen Abscheider mit einem Einlass zum Einführen der Suspension biologischer Zellen in den Abscheider und einen ersten Auslass zum Strömen des Zellkonzentrats aus dem Abscheider,
ii. eine Hardwarekomponente, umfassend eine erste Pumpe zum Strömen der Suspension biologischer Zellen zum Einlass des Abscheiders, eine zweite Pumpe zum Strömen des Zellkonzentrats von einem ersten Auslass des Abscheiders, einen ersten optischen Sensor, der dem ersten Auslass zugeordnet ist, einen zweiten optischen Sensor, der dem zweiten Auslass zugeordnet ist, und eine programmierbare Steuerung, die konfiguriert ist zum

a. Einstellen einer Ziel-Auslasskonzentration und Einstellen eines Zielverhältnisses zwischen Einlasskonzentration und Auslasskonzentration;
b. Bestimmen einer Einlassrate und einer Einlasskonzentration;
c. Berechnen einer Anfangsauslassrate, anhand des Zielverhältnisses, der gemessenen Einlassrate und der gemessenen Einlasskonzentration;
d. Einstellen der Anfangsauslassrate für den Abscheider und Betreiben des Abscheiders mit dieser Auslassrate;
e. Durchführen einer optischen Messung an dem ersten und zweiten optischen Sensor;
f. Vergleichen des Verhältnisses zwischen der optischen Messung an dem ersten optischen Sensor und der optischen Messung an dem zweiten optischen Sensor mit dem Zielverhältnis;
g. Anpassen des Abscheiders an eine angepasste Auslassrate durch Anpassen der zweiten Pumpe, wenn die Verhältnisse nicht gleich sind; und
h. Durchführen der Schritte e-g, bis das Verhältnis zwischen der optischen Messung an dem ersten optischen Sensor und der optischen Messung an dem zweiten optischen Sensor gleich dem Zielverhältnis ist, wodurch die Zielaustrittszellkonzentration produziert wird.

**Revendications**

**1.** Procédé ex-vivo pour la séparation d'une suspension de cellules biologiques à une concentration de cellule de sortie cible au moyen d'un système comprenant un séparateur avec une entrée pour l'introduction de la suspension de cellules biologiques dans le séparateur et une première sortie pour l'écoulement de concentré de cellule à partir du séparateur, un premier capteur optique sur l'entrée, un second capteur optique sur la première sortie, au moins une pompe associée au séparateur, et un contrôleur, le procédé comprenant :

a. la définition d'une concentration de sortie cible et d'un rapport cible entre de la concentration d'entrée et la concentration de sortie ;
b. la détermination d'un taux d'entrée et d'une concentration d'entrée ;
c. le calcul d'un taux de sortie initial sur la base du rapport cible, du taux d'entrée mesuré et de la concentration d'entrée mesurée ;
d. le réglage du taux de sortie initial pour le séparateur et l'actionnement du séparateur à ce taux de sortie ;
e. la réalisation d'une mesure optique au niveau des premier et second capteurs optiques ;
f. la comparaison du rapport entre la mesure optique au niveau du premier capteur optique et la mesure optique au niveau du second capteur optique au rapport cible ;
g. le réglage du séparateur à un taux de sortie réglé si les rapports ne sont pas égaux par le réglage de l'au moins une pompe associée au séparateur ; et

**EP 4 309 693 B1**

h. la réalisation des étapes e- g jusqu'à ce que le rapport entre la mesure optique au niveau du premier capteur optique et la mesure optique au niveau du second capteur optique soit égal au rapport cible, produisant la concentration de cellule de sortie cible.

2. Procédé de séparation selon la revendication 1, dans lequel le séparateur est un séparateur à membrane tournante.

3. Procédé de séparation selon la revendication 2, dans lequel le séparateur comprend une première et une seconde sortie, la première sortie pour l'écoulement d'un rétentat à partir du séparateur.

4. Procédé de séparation selon la revendication 1, dans lequel le séparateur comprend une chambre centrifuge.

5. Procédé de séparation selon l'une quelconque des revendications précédentes, dans lequel la mesure optique est une densité optique.

6. Procédé de séparation selon l'une quelconque des revendications précédentes, dans lequel le réglage de l'au moins une pompe associée au séparateur comprend la modification d'une pompe associée à la première sortie pour régler le taux de sortie.

7. Procédé de séparation selon l'une quelconque des revendications précédentes, dans lequel le réglage comprend le calcul de la différence entre le taux de sortie mesuré et le taux de sortie initial et l'ajout de la différence au taux de sortie initial pour obtenir le taux de sortie réglé.

8. Procédé ex-vivo pour la séparation d'une suspension de cellules biologiques à une concentration de cellule de sortie cible au moyen d'un système comprenant un séparateur avec une entrée pour l'introduction de la suspension de cellules biologiques dans le séparateur et une première sortie pour l'écoulement de concentré de cellule à partir du séparateur, un premier capteur optique sur l'entrée, un second capteur optique sur la première sortie, au moins une pompe associée au séparateur, et un contrôleur, le procédé comprenant :

a. la définition d'une concentration de sortie cible et d'un rapport cible entre la concentration de sortie et de la concentration d'entrée et ;
b. la détermination d'un taux de sortie et d'une concentration d'entrée ;
c. le calcul d'un taux d'entrée initial sur la base du rapport cible, du taux de sortie mesuré et de la concentration d'entrée mesurée ;
d. le réglage du taux d'entrée initial pour le séparateur et l'actionnement du séparateur à ce taux d'entrée ;
e. la réalisation d'une mesure optique au niveau des premier et second capteurs optiques ;
f. la comparaison du rapport entre la mesure optique au niveau du premier capteur optique et la mesure optique au niveau du second capteur optique au rapport cible ;
g. le réglage du séparateur à un taux d'entrée réglé si les rapports ne sont pas égaux par le réglage de l'au moins une pompe associée au séparateur ; et
h. la réalisation des étapes e-g jusqu'à ce que le rapport entre la mesure optique au niveau du second capteur optique et la mesure optique au niveau du premier capteur optique soit égal au rapport cible, produisant la concentration de cellule de sortie cible.

9. Procédé de séparation selon la revendication 8, dans lequel le séparateur est un séparateur à membrane tournante.

10. Procédé de séparation selon la revendication 9, dans lequel le séparateur comprend une première et une seconde sortie, la première sortie pour l'écoulement d'un rétentat à partir du séparateur.

11. Procédé de séparation selon la revendication 8, dans lequel le séparateur comprend une chambre centrifuge.

12. Procédé de séparation selon l'une quelconque des revendications 8 à 11, dans lequel la mesure optique est une densité optique.

13. Procédé de séparation selon l'une quelconque des revendications 8 à 12, dans lequel le réglage de l'au moins une pompe associée au séparateur comprend la modification d'une pompe associée à la première entrée pour régler le taux d'entrée.

14. Procédé de séparation selon l'une quelconque des revendications 8 à 13, dans lequel le réglage comprend le calcul de la différence entre le taux d'entrée mesuré et le taux d'entrée initial et l'ajout de la différence au taux d'entrée initial pour obtenir le taux d'entrée réglé.

15. Système de séparation d'une suspension de cellules biologiques comprenant :

i. un circuit de fluide comprenant un séparateur avec une entrée pour l'introduction de la suspension de cellules biologiques dans le séparateur et une première sortie pour l'écoulement d'un concentrat de cellules à partir du séparateur ;
ii. un composant matériel comprenant une première pompe pour l'écoulement de la suspension de cellules biologiques vers l'entrée du séparateur, une seconde pompe pour l'écoulement du concentré de cellules à partir d'une première sortie du séparateur, un premier capteur optique associé à la première sortie, un second capteur optique associé à la seconde sortie, et un contrôleur programmable configuré pour

a. définir une concentration de sortie cible et définir un rapport cible entre de la concentration d'entrée et la concentration de sortie ;
b. déterminer un taux d'entrée et une concentration d'entrée ;
c. calculer un taux de sortie initial sur la base du rapport cible, du taux d'entrée mesuré et de la concentration d'entrée mesurée ;
d. définir le taux de sortie initial pour le séparateur et actionner le séparateur à ce taux de sortie ;
e. réaliser une mesure optique au niveau des premier et second capteurs optiques ;
f. comparer le rapport entre la mesure optique au niveau du premier capteur optique et la mesure optique au niveau du second capteur optique au rapport cible ;
g. régler le séparateur à un taux de sortie réglé par le réglage de la seconde pompe si les rapports ne sont pas égaux ; et
h. réaliser les étapes e- g jusqu'à ce que le rapport entre la mesure optique au niveau du premier capteur optique et la mesure optique au niveau du second capteur optique soit égal au rapport cible, produisant la concentration de cellule de sortie cible.

**FIG. 1A**

**FIG. 1B**

FIG. 2

**FIG. 3**

**FIG. 4**

FIG. 5

300 — Microprocessor (304), Memory (306)

302 — Input

Scale(s)

Sensors S1 & S2

Pump(s)

Clamp(s)

Spinning membrane drive Or Centrifugal separator

308 — Video display

**Set target concentration ratio**

$$\frac{C_{in}}{C_{out}} = \frac{OD_{in}}{OD_{out}} = \frac{Target}{concentration\ ratio\ (TCR)}$$

**Set Initial outlet rate**

$$Q_{out\text{-}initial} = TCR \cdot Q_{in}$$

**Measured optical densities with sensors**

$$\frac{OD_{in\text{-}measured}}{OD_{out\text{-}measured}}$$

$$\frac{OD_{in\text{-}measured}}{OD_{out\text{-}measured}} = TCR$$

**Yes** → Continue procedure

**No**

Wait pre-determined time

**Calculate measured outlet rate**

$$Q_{out\text{-}measured} = \frac{OD_{in\text{-}measured}}{OD_{out\text{-}measured}} Q_{in}$$

**Calculate outlet rate inaccuracy**

$$Q_{out\text{-}diff} = Q_{out\text{-}initial} - Q_{out\text{-}measured}$$

**Update outlet rate**

$$Q_{out\text{-}adjusted} = Q_{out\text{-}initial} + Q_{out\text{-}diff}$$

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63391415
- US 5194145 A
- US 2018133655 A1
- US 8075468 B
- US 5053121 A
- US 5868696 A
- US 8556793 B